# EUROPÄISCHE PATENTANMELDUNG

(11) **EP 2 045 278 A1**
(43) Veröffentlichungstag der Anmeldung: **08.04.2009**
(21) Anmeldenummer: 07019525.0
(22) Anmeldetag: 05.10.2007
(51) Int. Cl.: C08G 18/08, A61L 15/26, A61L 15/42

(54) **Verfahren zur Herstellung von Polyurethan-Schäumen**

(71) Anmelder: Bayer MaterialScience AG, 51368 Leverkusen (DE); Bayer Innovation GmbH, 40225 Düsseldorf (DE)
(72) Erfinder: Schönberger, Jan, Dr., 42655 Solingen (DE); Mager, Michael, Dr., 51375 Leverkusen (DE); Rische, Thorsten, Dr., 59423 Unna (DE); Dörr, Sebastian, Dr., 40597 Düsseldorf (DE); Feller, Thomas, 42659 Solingen (DE); Heckes, Michael, 47800 Krefeld (DE); Dietze, Melita, 40699 Erkrath (DE); Fugmann, Burkhard, Dr., 40878 Ratingen (DE)
(74) Vertreter: Marx, Thiemo Patrick

(57) **Zusammenfassung**

Die Erfindung betrifft ein Verfahrung zur Herstellung von Polyurethan-Schäumen, durch Aufschäumen und Trocknen von Mischungen spezieller Polyurethan-Dispersionen mit Vernetzern.

## Beschreibung

Die Erfindung betrifft ein Verfahrung zur Herstellung von Polyurethan-Schäumen, durch Aufschäumen und Trocknen von Mischungen spezieller Polyurethan-Dispersionen mit Vernetzern.

In der Wundbehandlung ist die Verwendung von Polyurethan-Schäumen als Wundauflage seit langem bekannt. Um eine gute Absorption von Wundflüssigkeit zu gewährleisten, werden dazu in der Regel hydrophile Polyurethan-Schäume eingesetzt. Diese werden erhalten durch Umsetzung von Mischungen aus Diisocyanaten und Polyolen bzw. NCO-funktionellen Polyurethan-Prepolymeren mit Wasser in Gegenwart bestimmter Katalysatoren sowie (Schaum-) Additiven. In der Regel werden hierbei aromatische Diisocyanate eingesetzt, da sich diese am besten verschäumen lassen. Zahlreiche Ausführungsformen dieser Verfahren sind bekannt, beispielsweise beschrieben in US 3,978,266, US 3,975,567 und EP-A 0 059 048. Die vorgenannten Verfahren weisen jedoch den Nachteil auf, dass reaktive Mischungen eingesetzt werden müssen, enthaltend Diisocyanate oder entsprechende Prepolymere, deren Handhabung technisch aufwendig ist, da z.B. entsprechende Schutzmaßnahmen erforderlich sind.

Es ist darüber hinaus bekannt, Schäume aus Polyurethan-Dispersionen herzustellen, indem man in Gegenwart geeigneter (Schaum-) Additive durch kräftiges Rühren Luft einträgt. Nach dem Trocknen und Aushärten werden so genannte mechanische Polyurethan-Schäume erhalten. Solche Schäume sind im Zusammenhang mit Wundauflagen in EP-A 0 235 949 und EP-A 0 246 723 beschrieben, wobei dem Schaum entweder ein selbst-haftendes Polymer zugesetzt oder der Schaum auf einen Film eines selbst-haftenden Polymers aufgebracht wird. Die Verwendung der Schäume als solche, d.h. ohne selbst-haftende Polymere wird nicht beschrieben. Die in EP-A 0 235 949 und EP-A 0 246 723 aufgeführten Beispiele beschreiben darüber hinaus zwingend die Verwendung von Polyaziridinen als Vernetzer, die nach heutigem Stand des Wissens aufgrund ihrer Toxizität nur noch begrenzt eingesetzt werden sollten. In US 4,655,210 ist die Verwendung der vorgenannten mechanischen Schäume für Wundauflagen beschrieben, die einen speziellen Aufbau aus Träger, Schaum und Hautkontakt-Schicht aufweisen.

Die in EP-A 0 235 949, EP-A 0 246 723 und US 4,655,210 beschriebenen Polyurethan-Dispersionen werden durch den Einbau von bestimmten Carbonsäuren wie Dimethylolcarbonsäuren und Neutralisation der Carbonsäuren mit tertiären Aminen, beispielsweise Triethylamin, anionisch hydrophiliert. Die so gebildeten Ammoniumcarboxylate sind jedoch zersetzlich, insbesondere bei höheren Temperaturen, wodurch die Amine wieder freigesetzt werden. Dies ist bei der Verarbeitung solcher Produkte und besonders in Hautkontakt von großem Nachteil. Bei der Herstellung dieser Polyurethan-Dispersionen wurden die Dimethylolcarbonsäuren darüber hinaus gelöst eingesetzt, z.B. in Dimethylformamid oder N-Methylpyrrolidon, wodurch die fertigen Produkte insgesamt einen hohen Gehalt an gesundheitlich bedenklichen, flüchtigen organischen Bestandteilen (VOC) aufweisen, im Falle des eingesetzten Witcobond^{™} 290 H 10,8 g/Liter (ohne Wasser).

EP-A 0 760 743 beschreibt darüber hinaus solche mechanischen Schäume auf Basis von Latex-Dispersionen, die jedoch nicht aus Polyurethanen bestehen und schlechtere mechanische Eigenschaften aufweisen.

Aufgabe der vorliegenden Erfindung war daher die Bereitstellung von neuen Wundauflagen auf Basis von Polyurethanen, welche auf möglichst einfache Weise und ohne die Verwendung gesundheitsschädlicher Aufbaukomponenten oder Additive zugänglich sind. Weitere Voraussetzung ist, dass diese Wundauflagen gute mechanische Eigenschaften, eine hohe Aufnahmekapazität von physiologischer Salzlösung sowie eine hohe Wasserdampfdurchlässigkeit aufweisen. Ferner sollen die Schäume eine zufriedenstellende Wasserbeständigkeit aufweisen.

Es wurde nun gefunden, dass solche Wundauflagen auf Polyurethanbasis zugänglich sind, indem Zusammensetzungen, die spezielle wässrige Polyurethan-Dispersionen sowie Vernetzer enthalten, aufgeschäumt und anschließend unter zumindest teilweiser Vernetzung getrocknet werden.

Gegenstand der vorliegenden Erfindung ist daher ein Verfahren zur Herstellung von geschäumten Artikeln, bevorzugt Wundauflagen, bei dem Zusammensetzungen enthaltend mittels Sulfonatgruppen anionisch hydrophilierte, wässrige Polyurethan-Dispersionen (I) zusammen mit Vernetzern (II) aufgeschäumt und unter zumindest teilweiser chemischer Vernetzung getrocknet werden.

Unter Vernetzung wird im Sinne der vorliegenden Erfindung die Bildung kovalenter Bindungen zwischen reaktiven Gruppen des Vernetzers und den in den Polyurethandispersionen enthaltenen Polyurethanen verstanden.

Wundauflagen aus Polyurethan-Schäumen im Sinne der Erfindung sind poröse Materialien, bevorzugt mit zumindest teilweise vorhandener Offenzelligkeit, welche im wesentlichen aus Polyurethanen bestehen und Wunden im Sinne einer sterilen Abdeckung vor Keimen bzw. Umgebungseinflüssen schützen, eine schnelle und hohe Absorption von physiologischer Salzlösung bzw. Wundflüssigkeit aufweisen, durch geeignete Feuchtdurchlässigkeit für ein geeignetes Wundklima sorgen und eine ausreichende mechanische Festigkeit aufweisen.

Bevorzugt besitzen diese Dispersionen zur anionischen Hydrophilierung ausschließlich Sulfonatgruppen.

Bevorzugt weisen die speziellen Polyurethan-Dispersionen (I) einen niedrigen Grad an hydrophilen anionischen Gruppen, bevorzugt von 0,1 bis 15 Milliequivalenten pro 100g Polyurethan (Festharz) auf.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie.

Bevorzugt besitzen die Polyurethan-Dispersionen (I) Feststoffgehalte von 30 bis 70 Gew.-%, besonders bevorzugt 50 bis 70 Gew.-%, ganz besonders bevorzugt 55 bis 65 Gew.-% und insbesondere 60 bis 65 Gew.-%, bezogen auf das darin enthaltene Polyurethan.

Bevorzugt weisen diese Polyurethan-Dispersionen weniger als 0,5 Gew.-%, besonders bevorzugt weniger als 0,2 Gew.-%, bezogen auf die gesamte Dispersionen, an ungebundenen organischen Aminen auf.

Solche bevorzugt einzusetzenden Polyurethan-Dispersionen (I) sind erhältlich, in dem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   A3) gegebenenfalls hydroxyfunktionellen Verbindungen mit Molekulargewichten von 62 bis 399 g/mol sowie
   A4) gegebenenfalls isocyanatreaktiven, anionischen oder potentiell anionischen und/oder gegebenenfalls nichtionischen Hydrophilierungsmitteln,
   hergestellt, und
B) dessen freie NCO-Gruppen dann ganz oder teilweise
   B1) gegebenenfalls mit aminofunktionellen Verbindungen mit Molekulargewichten von 32 bis 400 g/mol und
   B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden.

Falls gewünscht, kann das Prepolymer vor, während oder nach der Dispergierung durch Beimischung einer Base ganz oder teilweise in die anionische Form überführt werden.

Um eine anionische Hydrophilierung zu erreichen müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO⁻ oder - SO₃⁻ oder -PO₃²⁻ als anionische bzw. deren ganz oder teilweise protonierte Säureformen als potentiell anionische Gruppen aufweisen.

Bevorzugt werden in A4) und/oder B2) solche Verbindungen zur anionischen oder potentiell anionischen Hydrophilierung eingesetzt, die als anionische oder potentiell anionische Funktionalität ausschließlich Sulfonsäure- bzw. Sulfonatgruppen (-SO₃H bzw. -SO₃M, mit M = Alkali- oder Erdalkalimetall) aufweisen.

Geeignete Polyisocyanate der Komponente A1) sind die dem Fachmann an sich bekannten aliphatischen oder cycloaliphatischen Polyisocyanate einer NCO-Funktionalität von größer oder gleich 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts, 1,4-Cydohexylendiisocyanat, 4-Isocyanatomethyl-1,8-octandiisocyanat (Nonantriisocyanat), sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥ 2 aufweisen, mit Uretdion-, Isocyanurat-, Urethan-, Allophanat-, Biuret-, Iminooxadiazindion- oder Oxadiazintrionstruktur sowie Mischungen aus diesen anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt 2 bis 2,6 und besonders bevorzugt 2 bis 2,4.

Besonders bevorzugt werden in A1) Hexamethylendiisocyanat, Isophorondiisocyanat oder die isomeren Bis-(4,4'-isocyanatocyclohexyl)methane sowie Mischungen der vorgenannten Diisocyanate eingesetzt.

In A2) werden polymere Polyole mit einem zahlenmittleren Molekulargewicht Mₙ von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbemsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) hydroxylgruppenaufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Hydroxylgruppen aufweisende Polycarbonate sind bevorzugt linear gebaut.

Ebenfalls können in A2) Polyetherpolyole eingesetzt werden.

Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxy-capronsäureester, ω-Hydroxyhexyl-γ-hydroxybuttersäure-ester, Adipinsäure-(β-hydroxyethyl)ester oder Terephthalsäurebis(β-hydroxyethyl)-ester.

Ferner können in A3) auch monofunktionelle isocyanatreaktive Hydroxyl-gruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykolmonomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Geeignete anionisch hydrophilierende Verbindungen der Komponente A4) sind Salze der Mono- und Dihydroxysulfonsäuren. Beispiele solcher anionischen Hydrophilierungsmittel sind das Addukt von Natriumbisulfit an Buten-2-diol-1,4, wie es in DE-A 2 446 440, Seite 5 - 9, Formel I-III beschrieben ist.

Geeignete nichtionisch hydrophilierende Verbindungen der Komponente A4) sind z.B. Polyoxyalkylenether, die mindestens eine Hydroxy-, Amino- oder Thiolgruppe enthalten. Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentämethylendiamin, Diethylentriamin, 4,4-Diaminodicyclohexylmethan und/oder Dimethylethylendiamin eingesetzt werden.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)-aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Geeignete anionisch hydrophilierende Verbindungen der Komponente B2) sind Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiaminpropyl- oder -butylsulfonsäure, 1,2- oder 1,3-Propylendiamin-β-ethylsulfonsäure oder Taurin. Weiterhin kann das Salz der Cyclohexylaminopropansulfonsäure (CAPS) aus WO-A 01/88006 als anionisches Hydrophilierungsmittel verwendet werden.

Besonders bevorzugte anionische Hydrophilierungsmittel B2) sind solche, die Sulfonat-gruppen als ionische Gruppen und zwei Aminogruppen enthalten, wie die Salze der 2-(2-Aminoethylamino)ethylsulfonsäure und 1,3-Propylendiamin-β-ethylsulfonsäure.

Zur Hydrophilierung können auch Mischungen aus anionischen und nichtionischen Hydrophilierungsmitteln verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten A4) und B2), wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und B1)
0,1 bis 15 Gew.-% Summe der Komponenten Komponente A4) und B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und B1)
0,1 bis 13,5 Gew.-% Summe der Komponenten A4) und B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der speziellen Polyurethan-Dispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren verfahren.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanat-Gruppen zu isocyanatreaktiven Gruppen 1,05 bis 3,5, bevorzugt 1,1 bis 3,0, besonders bevorzugt 1,1 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss wird in einem weiteren Verfahrensschritt, falls noch nicht oder nur teilweise geschehen, das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers umgesetzt. Bevorzugt wird die Kettenverlängerung/-terminierung vor der Dispergierung in Wasser durchgeführt.

Geeignete Komponenten zur Kettenverlängerung sind organische Di- oder Polyamine B1) wie beispielsweise Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen B1), die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- methylaminobutan, Alkanolamine wie N-Aminoethylethanolamin, Ethanolamin, 3-Aminopropanol, Neopentanolamin zur Kettenverlängerung bzw. -terminierung eingesetzt werden.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin verwendet.

Werden zur Kettenverlängerung anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung und Kettenterminierung eingesetzten Verbindungen zu freien NCO-Gruppen des Prepolymers, liegt zwischen 40 und 150 %, bevorzugt zwischen 50 und 120 %, besonders bevorzugt zwischen 60 und 120 %.

Die aminischen Komponenten B1) und B2), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den erfindungswesentlichen Dispersionen beträgt typischerweise weniger als 1 Gew.-%, bevorzugt weniger als 0,5 Gew.-% bezogen auf die gesamte Dispersion.

Der pH-Wert der erfindungswesentlichen Dispersionen beträgt typischerweise weniger als 8,0, bevorzugt weniger als 7,5 und liegt besonders bevorzugt zwischen 5,5 und 7,5.

Als Vernetzer (II) können grundsätzlich alle organischen, mindestens difunktionellen Verbindungen eingesetzt werden, welche unter den angegebenen Trocknungsbedingungen kovalente Bindungen mit dem eingesetzten Polyurethan der Polyurethan-Dispersion (I) bilden, und somit zur gewünschten Verbesserung der mechanischen Eigenschaften und/oder der Wasserbeständigkeit führen. Beispiele für solche Vernetzer sind unblockierte, gegebenenfalls hydrophilierte Polyisocyanate, Amid- und Amin-Formaldehydharze, Phenolharze, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze und Anilinharze enthalten.

Bevorzugt werden als Vernetzer unblockierte Polyisocyanate oder Melaminharze eingesetzt, besonderes bevorzugt jedoch unblockierte Polyisocyanate, ganz besonders bevorzugt, hydrophilierte Polyisocyanate, welche sich nach allen gängigen Misch- und Dispergiertechniken besonders leicht in die Polyurethan-Dispersion (I) einarbeiten lassen.

Möglich ist auch die Verwendung von Mischungen verschiedener Vernetzer der Komponente (II)..

Neben den Dispersionen (I) und den Vernetzern (II) können die aufzuschäumenden Zusammensetzungen auch Hilfs- und Zusatzstoffe (III) enthalten.

Beispiele für solche Hilfs- und Zusatzstoffe (III) sind Schaumhilfsmittel wie Schaumbildner und -stabilisatoren, Verdicker bzw. Thixotropiermittel, Antioxidantien, Lichtschutzmittel, Emulgatoren, Weichmacher, Pigmente, Füllstoffe und Verlaufshilfsmittel.

Bevorzugt sind als Hilfs- und Zusatzstoffe (III) Schaumhilfsmittel wie Schaumbildner und - stabilisatoren enthalten. Geeignet sind handelsübliche Verbindungen wie Fettsäureamide, , Kohlenwasserstoffsulfonate, -sulfate oder Fettsäuresalze, wobei der lipophile Rest bevorzugt 12 bis 24 Kohlenstoffatome enthält, sowie Alkylpolyglycoside, die nach dem Fachmann an sich bekannten Methoden durch Umsetzung von längerkettigen Monoalkoholen (4 bis 22 C-Atome im Alkylrest), mit Mono-, Di- oder Polysacchariden erhältlich sind (siehe z.B. Kirk-Othmer, Encyclopedia of Chemical Technology, John Wiley & Sons, Vol. 24, S. 29).

Besonders geeignete Schaumhilfsmittel sind EO/PO-Blockcopolymere, die nach dem Fachmann an sich bekannten Methoden durch Addition von Ethylenoxid und Propylenoxid an OH- oder NH-funktionelle Startermoleküle erhältlich sind (siehe z.B. Kirk-Othmer, Encyclopedia of Chemical Technology, John Wiley & Sons, Vol. 24, S. 28). Zur Verbesserung der Schaumbildung, Schaumstabilität oder der Eigenschaften des resultierenden PolyurethanSchaums, können neben den EO/PO-Blockcopolymeren noch weitere Additive in der Komponente (III) enthalten sein. Solche weiteren Additive können grundsätzlich alle an sich bekannten anionischen, nichtionischen und kationischen Tenside sein. Bevorzugt werden jedoch allein die EO/PO-Blockcopolymere als Komponente (III) eingesetzt.

Als Verdicker können handelsübliche Verdicker eingesetzt werden, wie Dextrin-, Stärke-oder Cellulosederivate, z.B. Celluloseether oder Hydroxyethylcellulose, Polysaccharidderivate wie Gummi arabicum, organische vollsynthetische Verdicker auf Basis von Polyacrylsäuren, Polyvinylpyrrolidonen, Poly(meth)acrylverbindungen oder Polyurethanen (assoziative Verdicker) sowie anorganische Verdicker, wie Betonite oder Kieselsäuren.

Die erfindungswesentlichen Zusammensetzungen enthalten, bezogen auf Trockensubstanz, typischerweise 90 bis 99,9 Gewichtsteile der Polyurethan-Dispersion (I), 0,1 bis 10 Gewichtsteile des Vernetzers (II) und 0 bis 10 Gewichtsteile Schaumhilfsmittel (III). Bevorzugt enthalten die erfindungswesentlichen Zusammensetzungen, bezogen auf die Trockensubstanz, 87,5 bis 98,9 Gewichtsteile der Dispersion (I), 0,1 bis 5 Gewichtsteile des Vernetzers (II) und 1 bis 7,5 Gewichtsteile Schaumhilfsmittel (III), besonders bevorzugt 90,5 bis 97 Gewichtsteile der Dispersion (I), 0,5 bis 2 Gewichtsteile des Vernetzers (II) und 2,5 bis 7,5 Gewichtsteile Schaumhilfsmittel (bezogen auf die Trockensubstanz).

Das Aufschäumen im erfindungsgemäßen Verfahren geschieht durch mechanisches Rühren der Zusammensetzung bei hohen Drehzahlen durch Schütteln oder durch Entspannung eines Treibgases.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Der so erhaltene Schaum wird beim Aufschäumen oder unmittelbar danach auf ein Substrat aufgetragen oder in eine Form gegeben und getrocknet.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich. Der Auftrag sowie die Trocknung können jeweils diskontinuierlich oder kontinuierlich durchgeführt werden, bevorzugt ist jedoch gänzlich kontinuierliches Verfahren.

Als Substrate geeignet sind Papiere (z.B. ein Trennpapiere) oder Folien, die ein einfaches Ablösen der Wundauflage vor ihrem Einsatz zur Abdeckung einer verletzten Stelle ermöglichen.

Die Trocknung erfolgt in der Regel unter Verwendung von an sich bekannten Heiz- und Trockenapparaten, wie (Umluft-) Trockenschränken, Heißluft oder IR-Strahlern. typischerweise bei erhöhten Temperaturen von 30 bis 200°C, bevorzugt 100 bis 170 °C, besonders bevorzugt 110 bis 160 °C. Bevorzugt ist überdies eine mindestens zweistufige Trocknung beginnend bei Temperaturen von 110 bis 130 °C und mit anschließender weiterer Trocknung (Vernetzung) bei erhöhten Temperaturen von 130 bis 160 °C.

Während der Trocknung erfolgt ebenso die Bildung kovalenter Bindungen zwischen dem Vernetzer (II) und dem Polyurethan aus der Polyurethan-Dispersion (I). Dadurch wird eine verbesserte Wasserbeständigkeit und/oder eine Verbesserung der mechanischen Eigenschaften erreicht.

Ein weiterer Gegenstand sind die nach dem erfindungsgemäßen Verfahren erhältlichen Wundauflagen.

Die Wundauflagen haben vor ihrer Trocknung typischerweise Schaumdichten von 50 bis 800 g/Liter, bevorzugt 100 bis 500 g/Liter, besonders bevorzugt 100 bis 250 g/Liter (Masse aller Einsatzstoffe [in g] bezogen auf das Schaumvolumen von einem Liter).

Die Wundauflagen besitzen nach ihrer Trocknung eine mikroporöse, offenporige Struktur mit miteinander kommunizierenden Zellen. Die Dichte der getrockneten Schäume liegt dabei typischerweise unterhalb von 0,4 g/cm³, bevorzugt ist sie geringer als 0,35 g/cm³, besonders bevorzugt liegt sie bei 0,01 bis 0,3 g/cm³ und ganz besonders bevorzugt bei 0,1 bis 0,3 g/cm³.

Das Absorptionsvermögen gegenüber physiologischer Salzlösung beträgt bei den Polyurethan-Schäumen typischerweise 100 bis 1500 %, bevorzugt 300 bis 1500 %, besonders bevorzugt 300 bis 800 % (Masse der aufgenommenen Flüssigkeit bezogen auf die Masse des trockenen Schaums; Bestimmung nach DIN EN 13726-1, Teil 3.2). Die Durchlässigkeit gegenüber Wasserdampf beträgt typischerweise 2000 bis 8000 g/24 h * m², bevorzugt 2000 bis 5000 g/24 h * m², besonders bevorzugt 2000 bis 4000 g/24 h *m² (Bestimmung nach DIN EN 13726-2, Teil 3.2).

Die Polyurethan-Schäume weisen eine gute mechanische Festigkeit und hohe Elastizität auf. Typischerweise sind die Werte für die maximale Spannung größer als 0,2 N/mm² und die maximale Dehnung ist größer als 250 %. Bevorzugt ist die maximale Dehnung größer als 350 %, besonders bevorzugt größer als 400 % (Bestimmung nach DIN 53504).

Die Wundauflagen haben nach dem Trocknen typischerweise eine Dicke von 0,1 mm bis 50 mm, bevorzugt 0,5 mm bis 20 mm, besonders bevorzugt 1 bis 10 mm, ganz besonders bevorzugt 1 bis 5 mm.

Die Wundauflagen können überdies mit weiteren Materialien verklebt, laminiert oder beschichtet werden, beispielsweise auf Basis von Hydrogelen, (semi-) permeablen Folien, Beschichtungen, Hydrokolloiden oder anderen Schäumen.

Sofern es zweckdienlich ist, kann im erfindungsgemäßen Verfahren ein Schritt zur Sterilisation erfolgen. Ebenso ist es grundsätzlich möglich, nach dem erfindungsgemäßen Verfahren erhältlichen Wundauflagen nach deren Herstellung zu sterilisieren. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung beispielsweise durch Gammabestrahlung erfolgt.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Bevorzugte Wirkstoffe der vorgenannten Art sind solche aus der Gruppe der Antiseptika, Wachstumsfaktoren, Proteaseinhibitoren und nichtsteroidalen Antiphlogistika/Opiate.

In einer bevorzugten Ausführungsform der vorliegenden Erfindung umfasst der Wirkstoff ein antiseptisches Biguanid und/oder dessen Salz, vorzugsweise das Hydrochlorid.

Als Biguanide werden Verbindungen bezeichnet, die sich vom Biguanid (C₂H₇N₅) ableiten, insbesondere dessen Polymere. Antiseptische Biguanide sind solche Verbindungen, die eine antimikrobielle Wirkung aufweisen, also als Bakteriostatika oder vorzugsweise als Bakterizide wirken. Die Verbindungen weisen vorzugsweise eine breite Wirkung gegen viele Bakterien auf und können durch eine Wirkung gegen E. coli von mindestens 0,5 µg/ml, vorzugsweise mindestens 12 oder mindestens 25 µg/ml gekennzeichnet werden (minimal mikrobizide Konzentration oder MMK, gemessen im Suspensionsversuch).

Ein bevorzugtes antiseptisches Biguanid gemäß dieser Erfindung ist Poly(imino[iminocarbonyl]iminopolymethylen), wobei die Verwendung von Poly(hexamethylen)biguanid (PHMB), das auch als Polyhexanid bezeichnet wird, als antiseptisches Biguanid besonders bevorzugt ist.

Der Begriff "antiseptische Biguanide" gemäß dieser Erfindung beinhaltet auch Metaboliten und/oder Prodrugs der antiseptischen Biguaniden. Antiseptische Biguanide können dabei als Racemate oder reine Isoformen vorliegen.

Bevorzugt enthalten die geschäumten Artikel aus Polyurethan-Schäumen beziehungsweise die Zusammensetzungen gemäß der vorliegenden Erfindung antiseptisches Biguanid und/oder dessen Salz, vorzugsweise das Hydrochlorid, in einer Konzentration von 0,01 bis 20 Gew-%, wobei die Konzentration von 0,1 bis 5 Gew-% besonders vorteilhaft ist. Das Biguanid kann eine beliebige Molekulargewichtsverteilung aufweisen.

### Beispiele:

Sofern nicht abweichend gekennzeichnet, beziehen sich alle Prozentangaben auf das Gewicht.

Sofern nicht abweichend vermerkt, beziehen alle analytischen Messungen auf Temperaturen von 23°C.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

### Verwendete Substanzen und Abkürzungen:

- Diaminosulfonat:: NH₂-CH₂CH₂-NH-CH₂CH₂-SO₃Na (45 %ig in Wasser)
- Desmophen^{®} C2200:: Polycarbonatpolyol, OH-Zahl 56 mg KOH/g, zahlenmittleres Molekulargewicht 2000 g/mol (Bayer MaterialScience AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlenmittleres zahlenmittleres Molekulargewicht 1000 g/mol (BASF AG, Ludwigshafen, DE)
- Polyether LB 25:: monofunktioneller Polyether auf Ethylenoxid-/Propylen-oxidbasis zahlenmittleres Molekulargewicht 2250 g/mol, OH-Zahl 25 mg KOH/g (Bayer MaterialScience AG, Leverkusen, DE)
- Pluronic^{®} PE 6800:: EO/PO-Blockcopolymer (BASF AG, Ludwigshafen, DE)

Die Bestimmung der mittleren Teilchengrößen (angegeben ist das Zahlenmittel) der Polyurethan-Dispersionen erfolgte mittels Laserkorrelations-Spektroskopie (Gerät: Malvern Zetasizer 1000, Malver Inst. Limited).

### Beispiel 1: Polyurethan-Dispersion 1

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 25,1 g Ethylendiamin, 116,5 g Isophorondiamin, 61,7 g Diaminosulfonat und 1030 g Wasser innerhalb von 10 min zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 61 % |
| | |
| Partikelgröße (LKS): | 312 nm |
| | |
| Viskosität (Viskosimeter, 23°C): | 241 mPas |
| | |
| pH (23°C): | 6,02 |

### Beispiel 2: Polyurethan-Dispersion 2

223,7 g PolyTHF^{®} 2000, 85,1 g PolyTHF^{®} 1000, 172,6 g Desmophen^{®} C2200 und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht war. Das fertige Prepolymer wurde mit 1005 g Aceton gelöst und dabei auf 50°C abgekühlt und anschließend wurde eine Lösung aus 5,70 Ethylendiamin, 26,4 g Isophorondiamin, 9,18 g Diaminosulfonat und 249,2 g Wasser innerhalb von 10 min zudosiert. Die Nachrührzeit betrug 10 min. Dann wurde durch Zugabe von 216 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 63% |
| | |
| Partikelgröße (LKS): | 495 nm |
| | |
| Viskosität (Viskosimeter, 23°C): | 133 mPas |
| | |
| pH (23°C): | 6,92 |

### Beispiel 3: Polyurethan-Dispersion 3

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 36,9 g 1,4-Diaminobutan, 116,5 g Isophorondiamin, 61,7 g Diaminosulfonat und 1076 g Wasser innerhalb von 10 min zudosiert. Die Nachrührzeit betrug 10 min. Dann wurde durch Zugabe von 1210 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 59% |
| | |
| Partikelgröße (LKS): | 350 nm |
| | |
| Viskosität (Viskosimeter, 23°C): | 126 mPas |
| | |
| pH (23°C): | 7,07 |

### Beispiel 4: Polyurethan-Dispersion 4

201,3 g PolyTHF^{®} 2000, 76,6 g PolyTHF^{®} 1000, 155,3, g Desmophen^{®} C2200, 2,50 g 1,4-Butandiol und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht war. Das fertige Prepolymer wurde mit 1010 g Aceton gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 243 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 62% |
| | |
| Partikelgröße (LKS): | 566 nm |
| | |
| Viskosität (Viskosimeter, 23°C): | 57 mPas |
| | |
| pH (23°C): | 6,64 |

### Beispiel 5: Polyurethan-Dispersion 5

201,3 g PolyTHF^{®} 2000, 76,6 g PolyTHF^{®} 1000, 155,3 g Desmophen^{®} C2200, 2,50 g Trimethylolpropan und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht war. Das fertige Prepolymer wurde mit 1010 g Aceton gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 293 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 56% |
| | |
| Partikelgröße (LKS): | 440 nm |
| | |
| Viskosität (Viskosimeter, 23°C): | 84 mPas |
| | |
| pH (23°C): | 6,91 |

### Beispiel 6: Polyurethan-Dispersion 6

1072 g PolyTHF^{®} 2000, 407,6 g PolyTHF^{®} 1000, 827 g Desmophen^{®} C2200 und 48,1 g Polyether LB 25 wurden in einer Standard-Rührapparatur auf 70°C aufgeheizt. Anschließend wurde bei 70°C innerhalb von 5 min ein Gemisch aus 257,4 g Hexamethylendiisocyanat und 340 g Isophorondiisocyanat zugegeben und bei 120°C gerührt, bis der theoretische NCO-Wert erreicht war. Das fertige Prepolymer wurde mit 4820 g Aceton gelöst und dabei auf 50°C abgekühlt und anschließend wurde eine Lösung aus 27,3 g Ethylendiamin, 126,5 g Isophorondiamin, 67,0 g Diaminosulfonat und 1090 g Wasser innerhalb von 10 min zudosiert. Die Nachrührzeit betrug 10 min. Dann wurde durch Zugabe von 1180 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 60% |
| | |
| Partikelgröße (LKS): | 312 nm |
| | |
| Viskosität (Viskosimeter, 23°C): | 286 mPas |
| | |
| pH (23°C): | 7,15 |

### Vergleichsbeispiel 1

Polyurethan-Dispersion, nicht erfindungsgemäß (keine Sulfonat-Gruppen, sondern nur Hydrophilierung durch nicht-ionische Gruppen und Carboxylat-Gruppen)

Es wurde vorgegangen analog Beispiel 1, jedoch wurde das Diaminosulfonat äquimolar ersetzt durch eine carboxylatgruppenhaltige Komponente:

206,8 g PolyTHF^{®} 2000, 78,7 g PolyTHF^{®} 1000, 159,5 g Desmophen^{®} C2200 und 9,3 g Polyether LB 25 wurden in einer Standard-Rührapparatur auf 70°C aufgeheizt. Anschließend wurde bei 70°C innerhalb von 5 min ein Gemisch aus 49,7 g Hexamethylendiisocyanat und 65,6 g Isophorondiisocyanat zugegeben und bei 120°C gerührt, bis der theoretische NCO-Wert erreicht war. Das fertige Prepolymer wurde mit 1010 g Aceton gelöst und dabei auf 50°C abgekühlt und anschließend wurde eine Lösung aus 5,3 g Ethylendiamin, 24,4 g Isophorondiamin, 11,9 g KV 1386 (40 %ige wässrige Lösung des Natriumsalzes von N-(2-Aminoethyl)-β-alanin, BASF AG, Ludwigshafen, DE) und 204 g Wasser innerhalb von 10 min zudosiert. Die Nachrührzeit betrug 10 min. Dann wurde durch Zugabe von 235 g Wasser dispergiert. Es folgte die Entfernung des Lösemittels durch Destillation im Vakuum. Dabei mussten aufgrund der hohen Viskosität noch insgesamt 250 g Wasser zugesetzt werden

Die erhaltene weiße Dispersion hatte nachfolgenden Eigenschaften:

| | |
|---|---|
| Feststoffgehalt: | 47 % |
| | |
| Partikelgröße (LKS): | 918 nm |
| | |
| Viskosität (Viskosimeter, 23°C): | 162 mPas |
| | |
| pH (23°C): | 7,22 |

Aufgrund der vergleichsweise hohen mittleren Teilchengröße von > 900 nm war im Gegensatz zu den rein sulfonathydrophilierten Dispersionen eine innerhalb weniger Tage einsetzende Sedimentation zu beobachten, nach welcher eine Weiterverarbeitung zu Schäumen erschwert war.

### Vergleichsbeispiel 2:

Polyurethan-Dispersion, nicht erfindungsgemäß (keine Sulfonat-Gruppen, sondern nur Hydrophilierung durch nicht-ionische Gruppen und Carboxylat-Gruppen)

Es wurde vorgegangen analog Vergleichsbeispiel 1, jedoch wurde die Menge der carboxylatgruppenhaltigen Hydrophilierungskomponente um 50 % erhöht (bei gleichem Grad der Kettenverlängerung).

206,8 g PolyTHF^{®} 2000, 78,7 g PolyTHF^{®} 1000, 159,5 g Desmophen^{®} C2200 und 9,3 g Polyether LB 25 wurden in einer Standard-Rührapparatur auf 70°C aufgeheizt. Anschließend wurde bei 70°C innerhalb von 5 min ein Gemisch aus 49,7 g Hexamethylendiisocyanat und 65,6 g Isophorondiisocyanat zugegeben und bei 120°C gerührt, bis der theoretische NCO-Wert erreicht war. Das fertige Prepolymer wurde mit 1010 g Aceton gelöst und dabei auf 50°C abgekühlt und anschließend wurde eine Lösung aus 5,3 g Ethylendiamin, 21,8 g Isophorondiamin, 17,9 g KV 1386 (40 %ige wässrige Lösung des Natriumsalzes von N-(2-Aminoethyl)-β-alanin, BASF AG, Ludwigshafen, DE) und 204 g Wasser innerhalb von 10 min zudosiert. Die Nachrührzeit betrug 10 min. Dann wurde durch Zugabe von 235 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 52,2 % |
| | |
| Partikelgröße (LKS): | 255 nm |
| | |
| Viskosität (Viskosimeter, 23°C): | 176 mPas |
| | |
| pH (23°C): | 8,31 |

Diese Polyurethan-Dispersion zeigt jetzt zwar im Unterschied zum Vergleichsbeispiel 2 eine geringere mittlere Teilchengröße, jedoch einen etwas höheren pH-Wert. Eine Weiterverarbeitung zu Schäumen war im Vergleich zu rein sulfonathydrophilierten Dispersionen deutlich schwieriger.

### Beispiele 6-8: Herstellung von vernetzten Schäumen, sowie Prüfung der Wasserbeständigkeit

Die in der Tabelle 1 angegebenen Mengen der Polyurethan-Dispersion 2 (Beispiel 2), des Schaumhilfsmittels Pluronic^{®} 6800 und des Vernetzers wurden vermischt und unter Verwendung eines handelsüblichen Handrührgerätes (Rührer aus gebogenem Draht) in 10 Minuten auf ein Schaumvolumen von 500 ml aufgeschlagen. Danach wurden die Schäume auf ein Trennpapier ausgestrichen (Nassfilmdicke: 4 mm). Die Schäume wurden 20 Min. bei 120 °C und 10 Min. bei 150 °C getrocknet. Es wurden durchweg reinweiße, hydrophile Schäume mit guten mechanischen Eigenschaften und einer feinen Porenstruktur erhalten.

Die vernetzten Schäume zeigten überdies eine gute Wasserbeständigkeit.

**Tabelle 1**

| | Menge [g] | | | | |
|---|---|---|---|---|---|
| Beispiel | Polyurethan-Dispersion 2 | Pluronic^{®} PE 6800 | Vernetzer | Wasserbeständigkeit⁴⁾ | Hydrophilie⁵⁾ |
| 7 | 120 | 13,3 | 0,76¹⁾ | gut | < 1 Sek. |
| 8 | 120 | 13,3 | 0,76²⁾ | gut | < 1 Sek. |
| 9 | 120 | 13,3 | 0,76³⁾ | gut | < 1 Sek. |

| | | | | | |
|---|---|---|---|---|---|
| ¹⁾ Acrafix ML (Hexamethoxymethylmelamin, Lanxess AG, Leverkusen DE); ²⁾ Bayhydur 305 (nicht-ionisch hydrophiliertes Polyisocyanat auf Basis Hexamethylendiisocyanat, NCO-Gehalt: 16,2 %, BayerMaterialScience AG, Leverkusen, DE); ³⁾ Bayhydur 3100 (nicht-ionisch hydrophiliertes Polyisocyanat auf Basis Hexamethylendiisocyanat, NCO-Gehalt: 17,4 %BayerMaterialScience AG, Leverkusen, DE); ⁴⁾ 18 h Lagerung eines 5x5 cm großen Schaums in destilliertem Wasser 37°C, danach vergleichende Prüfung der Weiterreißbeständigkeit (Klassifizierung: gering, mittel, gut); ⁵⁾ Zeit zur vollständigen Absorption eines Wassertropfens (als Maß für die Hydrophilie der Schäume) | | | | | |

### Vergleichsbeispiele 3: Herstellung eines unvernetzten Schaums, sowie Prüfung der Wasserbeständigkeit

In gleicher Weise wie in den Beispielen 7-9 beschrieben wurde ein unvernetzter Schaum hergestellt, d.h. auf die Verwendung eines Vernetzers wurde verzichtet. Der unvernetzte Schaum wies eine deutlich geringere Wasserbeständigkeit (Klassifizierung: "gering") als die vernetzten Schäume der Beispiele 7-9 auf.

## Patentansprüche

1. Verfahren zur Herstellung von geschäumten Artikeln, bei dem Zusammensetzungen enthaltend mittels Sulfonatgruppen anionisch hydrophilierte, wässrige Polyurethan-Dispersionen (I) sowie Vernetzer (II) aufgeschäumt und unter zumindest teilweiser chemischer Vernetzung getrocknet werden.

2. Verfahren gemäß Anspruch 1, **dadurch gekennzeichnet, dass** es sich bei den geschäumten Artikeln um Wundauflagen handelt.

3. Verfahren gemäß Anspruch 1 oder 2, **dadurch gekennzeichnet, dass** die Polyurethan-Dispersionen (I) zur anionischen Hydrophilierung ausschließlich Sulfonatgruppen enthalten.

4. Verfahren gemäß Anspruch 3, **dadurch gekennzeichnet, dass** die Sulfonatgruppen als Gegenionen Alkalimetallkationen besitzen.

5. Verfahren gemäß einem der Ansprüche 1 bis 4, **dadurch gekennzeichnet, dass** Polyurethan-Dispersionen (I) 0,1 bis 15 Milliequivalente pro 100 g Festharz an anionischen oder potentiell anionischen Gruppen bezogen auf Festharz aufweisen.

6. Verfahren gemäß einem der Ansprüche 1 bis 5, **dadurch gekennzeichnet, dass** die Dispersionen (I) Feststoffgehalte von 55 bis 65 Gew.-% bezogen auf das darin enthaltene Polyurethan aufweisen.

7. Verfahren gemäß einem der Ansprüche 1 bis 6, **dadurch gekennzeichnet, dass** die Dispersionen (I) erhältlich sind, indem
A) isocyanatfunktionelle Prepolymere aus
A1) organischen Polyisocyanaten
A2) polymeren Polyolen mit zahlenmittleren Molekulargewichten von 400 bis 8000 g/mol und OH-Funktionalitäten von 1,5 bis 6 und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und gegebenenfalls nichtionischen Hydrophilierungsmitteln,
hergestellt, und
B) deren freie NCO-Gruppen dann ganz oder teilweise
B1) gegebenenfalls mit aminofunktionellen Verbindungen mit Molekulargewichten von 32 bis 400 g/mol und
B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden.

8. Verfahren gemäß einem der Ansprüche 1 bis 7, **dadurch gekennzeichnet, dass** die aufzuschäumenden Zusammensetzungen als Vernetzer (II) unblockierte, gegebenenfalls hydrophilierte Polyisocyanate, Amid- und Amin-Formaldehydharze, Phenolharze, Aldehyd- und Ketonharze, Resole, Furanharze, Harnstoffharze, Carbamidsäureesterharze, Triazinharze, Melaminharze, Benzoguanaminharze, Cyanamidharze und Anilinharze enthalten.

9. Verfahren gemäß Anspruch 8, **dadurch gekennzeichnet, dass** als Vernetzer (II) unblockierte Polyisocyanate enthalten sind.

10. Verfahren gemäß Anspruch 9, **dadurch gekennzeichnet, dass** als Vernetzer (II) unblockierte, hydrophilierte Polyisocyanate enthalten sind.

11. Verfahren gemäß einem der Ansprüche 1 bis 10, **dadurch gekennzeichnet, dass** die aufzuschäumenden Zusammensetzungen als Hilfs- und Zusatzstoffe (III) Fettsäureamide, Sulfosuccinamide, Kohlenwasserstoffsulfonate, bzw. -sulfate, Alkylpolyglycoside, EO/PO-Blockcopolymere und/ oder Fettsäuresalze als Schaumbildner und -stabilisatoren enthalten.

12. Verfahren gemäß Anspruch 11, **dadurch gekennzeichnet, dass** als Schaumbildner und -stabilisatoren EO/PO-Blockcopolymere enthalten sind.

13. Verfahren gemäß einem der Ansprüche 1 bis 12, **dadurch gekennzeichnet, dass** Wirkstoffe aus der Gruppe der Antiseptika, Wachstumsfaktoren, Proteaseinhibitoren und nichtsteroidalen Antiphlogistika/Opiate mit verwendet werden.

14. Verfahren gemäß Anspruch 13, **dadurch gekennzeichnet, dass** als Wirkstoff ein antiseptisches Biguanid und/oder dessen Salz verwendet wird.

15. Geschäumte Artikel erhältlich nach einem Verfahren gemäß einem der Ansprüche 1 bis 14.

16. Geschäumte Artikel gemäß Anspruch 15, **dadurch gekennzeichnet, dass** diese eine mikroporöse, offenporige Struktur aufweisen und eine Dichte in getrocknetem Zustand von unterhalb 0,4 g/cm³ aufweisen.

17. Geschäumte Artikel gemäß Anspruch 13 oder 16, **dadurch gekennzeichnet, dass** diese ein Absorptionsvermögen gegenüber physiologischer Salzlösung von 100 bis 1500 % (Masse der aufgenommenen Flüssigkeit bezogen auf die Masse des trockenen Schaums; Bestimmung nach DIN EN 13726-1, Teil 3.2) und eine Durchlässigkeit gegenüber Wasserdampf von 2000 bis 8000 g/24 h * m² besitzen (Bestimmung nach DIN EN 13726-2, Teil 3.2).

18. Geschäumte Artikel gemäß einem der Ansprüche 13 bis 17, **dadurch gekennzeichnet, dass** diese auch einen Wirkstoff enthalten.

19. Geschäumte Artikel gemäß einem der Ansprüche 13 bis 18, **dadurch gekennzeichnet, dass** es sich um Wundauflagen handelt.

20. Zusammensetzungen enthaltend mittels Sulfonatgruppen anionisch hydrophilierte, wässrige Polyurethan-Dispersionen (I) sowie Vernetzer (II).

21. Zusammensetzungen gemäß Anspruch 20, **dadurch gekennzeichnet, dass** diese ferner Wirkstoffe aus der Gruppe der Antiseptika, Wachstumsfaktoren, Proteaseinhibitoren und nichtsteroidalen Antiphlogistika/Opiate umfassen.

22. Zusammensetzungen gemäß Anspruch 21, **dadurch gekennzeichnet, dass** als Wirkstoff ein antiseptisches Biguanid und/oder dessen Salz enthalten ist.
